(19) Europäisches Patentamt
European Patent Office
Office européen des brevets

(11) **EP 4 613 789 A1**

(12) **EUROPEAN PATENT APPLICATION**
published in accordance with Art. 153(4) EPC


(43) Date of publication:
**10.09.2025 Bulletin 2025/37**

(21) Application number: **23885684.3**

(22) Date of filing: **27.10.2023**

(51) International Patent Classification (IPC):
***C08G 59/24** (2006.01)* ***C07D 303/44** (2006.01)*
***C09D 7/63** (2018.01)* ***C09D 163/00** (2006.01)*
***C09J 11/06** (2006.01)* ***C09J 163/00** (2006.01)*

(52) Cooperative Patent Classification (CPC):
**C07D 303/44; C08G 59/24; C09D 7/63; C09D 163/00; C09J 11/06; C09J 163/00**

(86) International application number:
**PCT/JP2023/038956**

(87) International publication number:
**WO 2024/095928 (10.05.2024 Gazette 2024/19)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **04.11.2022 JP 2022177527**



(71) Applicant: **Daicel Corporation**
**Osaka-shi, Osaka 530-0011 (JP)**

(72) Inventors:
- **OKA, Mariko**
  **Tokyo 108-8230 (JP)**
- **TAKENAKA, Hiroto**
  **Tokyo 108-8230 (JP)**
- **SUZUKI, Hirose**
  **Tokyo 108-8230 (JP)**

(74) Representative: **Grünecker Patent- und Rechtsanwälte**
**PartG mbB**
**Leopoldstraße 4**
**80802 München (DE)**


(54) **EPOXY COMPOUND PRODUCT**


(57) Provided is a low-viscosity epoxy compound product that is not susceptible to curing shrinkage and can be used to form a cured product excelling in heat resistance and transparency. In the epoxy compound product according to the present disclosure, the purity of a compound represented by Formula (1) below is 90% or greater, and the total content proportion of a compound represented by Formula (a) below, a compound represented by Formula (b) below, a compound represented by Formula (c) below, and a compound represented by Formula (d) below is 10 mass% or less.

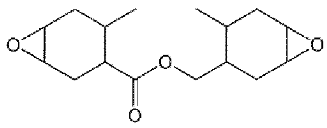

(1)

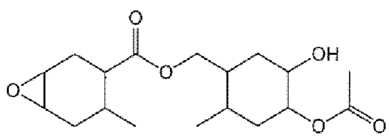

(a)


EP 4 613 789 A1



**(Cont. next page)**

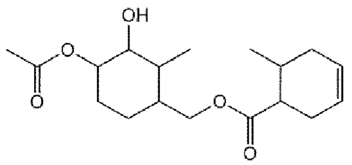

(b)

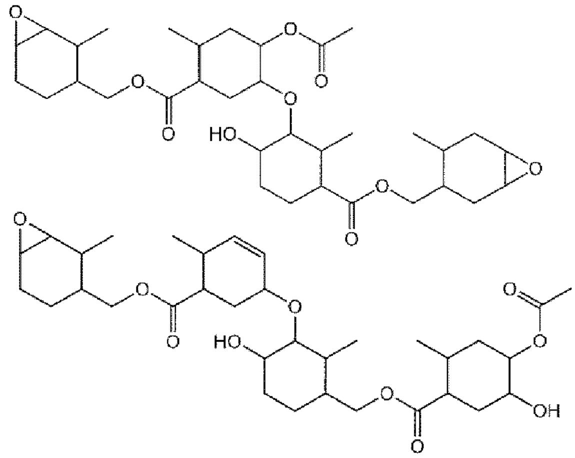

(c)

(d)

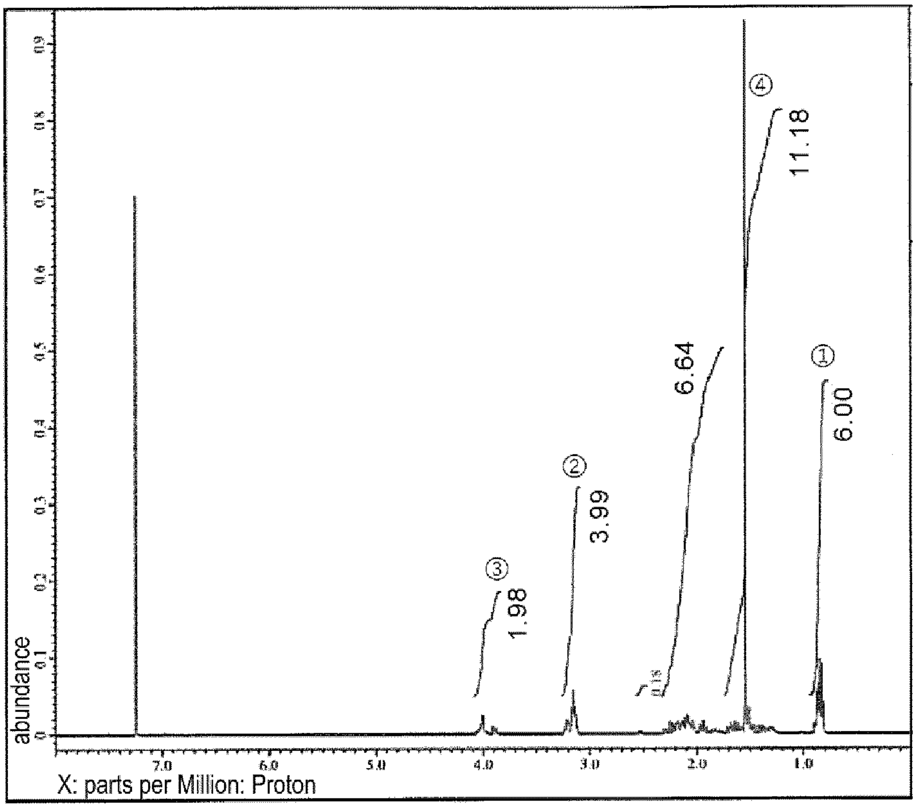
abundance
X: parts per Million: Proton
7.0
6.0
5.0
4.0
3.0
2.0
1.0
④
11.18
6.64
①
6.00
②
3.99
③
1.98

FIG. 1

## Description

Technical Field

The present disclosure relates to a high-purity epoxy compound product. The present application claims priority to JP 2022-177527 filed in Japan on November 4, 2022, the contents of which are incorporated herein by reference.

Background Art

A cured product having high strength and excelling in characteristics such as heat resistance and transparency can be formed by reacting an epoxy compound with various curing agents and curing catalysts. For example, alicyclic epoxy compounds having two or more epoxy groups are used as raw materials in encapsulating materials, coating agents, adhesives, inks, sealants, and the like.

Known examples of such alicyclic epoxy compounds include 3,4-epoxycyclohexylmethyl(3',4'-epoxy)cyclohexane carboxylate and 3,4-epoxy-6-methyl-cyclohexylmethyl(3',4'-epoxy-6'-methyl)cyclohexane carboxylate (see Patent Documents 1 and 2).

Citation List

Patent Document

Patent Document 1: WO 2019/138988
Patent Document 2: US 2890194

Summary of Invention

Technical Problem

In recent years, there has been a demand for curable compounds that are less susceptible to curing shrinkage. In addition, although 3,4-epoxy-6-methylcyclohexylmethyl(3',4'-epoxy-6'-methyl)cyclohexane carboxylate is relatively resistant to curing shrinkage, only products with low purity exist, and when such products are used, problems occur including poor heat resistance and transparency of the cured product and inferior handling properties due to the high viscosity.

Thus, an object of the present disclosure is to provide a low-viscosity epoxy compound product that is not susceptible to curing shrinkage and can be used to form a cured product excelling in heat resistance and transparency.

Solution to Problem

That is, the present disclosure provides an epoxy compound product in which the purity of a compound represented by Formula (1) below is 90% or greater; and

a total content proportion of a compound represented by Formula (a) below, a compound represented by Formula (b) below, a compound represented by Formula (c) below, and a compound represented by Formula (d) below is 10 mass% or less.

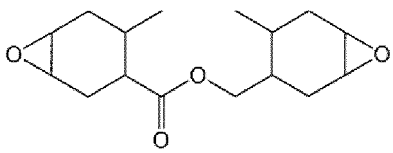

(1)

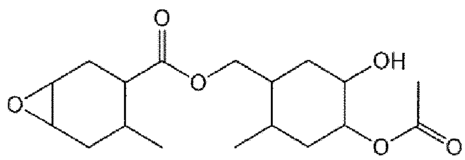

(a)

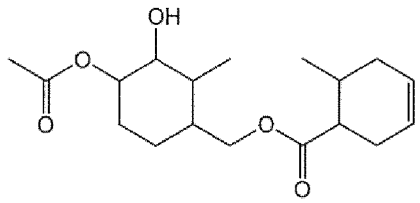

(b)

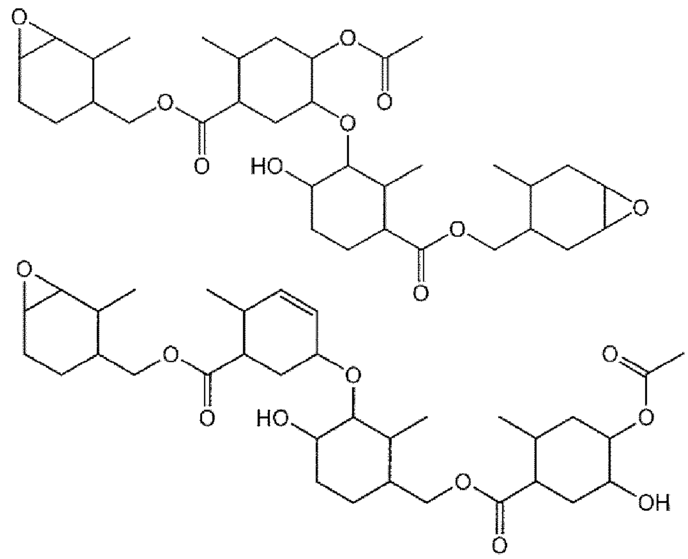

(c)

(d)

The abovementioned epoxy compound product preferably has a Hazen color number of 105 or less.

In the abovementioned epoxy compound product, the total content proportion of the compound represented by Formula (a) below, the compound represented by Formula (b) below, the compound represented by Formula (c) below, and the compound represented by Formula (d) may be 0.1 mass% or greater.

The present disclosure also provides a curable composition containing: the abovementioned epoxy compound product; and a curing agent and/or a curing catalyst.

The present disclosure also provides a curable composition containing: the abovementioned epoxy compound product; and another epoxy compound and/or an oxetane compound.

The abovementioned curable composition is preferably an adhesive, an encapsulant, or a coating agent.

The present disclosure also provides a cured product of the curable composition.

The present disclosure also provides an optical member provided with the cured product.

Moreover, the present disclosure provides a method for producing the abovementioned epoxy compound product by carrying out epoxidation, low-boiling fraction removal, and high-boiling fraction removal;

the epoxidation includes reacting 6-methyl-3-cyclohexenylmethyl(6'-methyl-3'-cyclohexenyl)carboxylate with an organic peracid to produce a reaction product;

the low-boiling fraction removal includes subjecting the reaction product to a low-boiling fraction removal treatment; and

the high-boiling fraction removal includes subjecting the reaction product to a high-boiling fraction removal treatment through thin film evaporation.

Advantageous Effects of Invention

The epoxy compound product of the present disclosure is not susceptible to curing shrinkage, excels in heat resistance and transparency, and has low viscosity.

Brief Description of Drawings

FIG. 1 is an $^{1}$H-NMR spectrum of an alicyclic epoxy compound product 1 produced in Example 1.

Description of Embodiments

Epoxy Compound Product

An epoxy compound product according to an embodiment of the present disclosure contains 3,4-epoxy-6-methyl-cyclohexylmethyl(3',4'-epoxy-6'-methyl)cyclohexane carboxylate (that is, a compound represented by Formula (1) below),

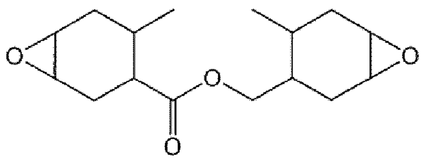

(1)

and the purity (or content proportion) of the compound is 90% or greater. From the perspective of being able to produce a cured product with lower viscosity and excellent transparency and particularly excelling in heat resistance and transparency, the purity of the compound represented by Formula (1) is preferably 91% or greater, more preferably 94% or greater, and even more preferably 96% or greater.

In addition, in the epoxy compound product, the total content proportion of a compound represented by Formula (a) below, a compound represented by Formula (b) below, a compound represented by Formula (c) below, and a compound represented by Formula (d) below is, in relation to the total amount (100 mass%) of the abovementioned epoxy compound product, 10 mass% or less, preferably 9 mass% or less, more preferably 6 mass% or less, and even more preferably 4 mass% or less. The total content proportion is, for example, 0.1 mass% or greater, and may be 0.2 mass% or greater, or 1 mass% or greater.

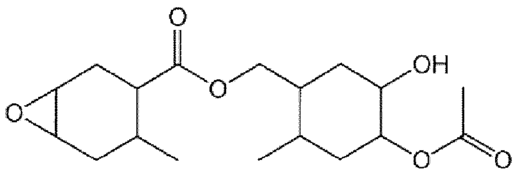

(a)

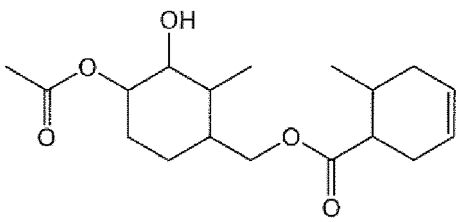

(b)

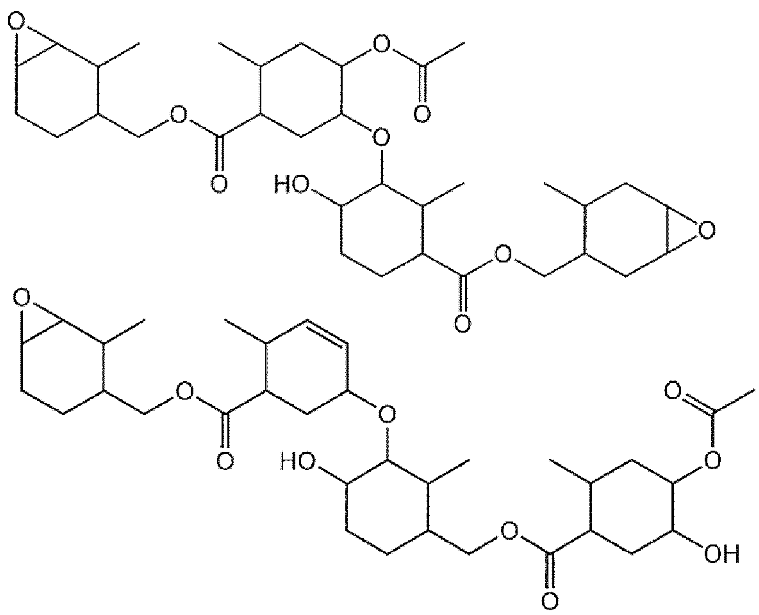

(c)

(d)

From the perspective of being able to produce a cured product with lower viscosity and excellent transparency and particularly excelling in heat resistance and transparency, in the epoxy compound product, even amongst impurities, the content proportion (total content proportion) of particularly compounds having a molecular weight of 100 or less and compounds having a molecular weight of 290 or greater (impurities including the compounds represented by Formulae (a) to (d)) is, in relation to the total amount (100 mass%) of the epoxy compound product, preferably 10 mass% or less, more preferably 5 mass% or less, even more preferably 3 mass% or less, and particularly preferably 2 mass% or less. Furthermore, the content proportion may be 0.1 mass% or greater.

The content proportion of the compound represented by Formula (a) is preferably 5 mass% or less, more preferably 3 mass% or less, and even more preferably 1 mass% or less. The content proportion of the compound represented by Formula (a) may be 0.1 mass% or greater. The content proportion of the compound represented by Formula (b) is preferably 1 mass% or less, more preferably 0.4 mass% or less, and even more preferably 0.2 mass% or less. The content proportion of the compound represented by Formula (b) may be 0.0001 mass% or greater. The content proportion of the compound represented by Formula (c) is preferably 5 mass% or less, more preferably 3 mass% or less, and even more preferably 1 mass% or less. The content proportion of the compound represented by Formula (c) may be 0.1 mass% or greater. The content proportion of the compound represented by Formula (d) is preferably 5 mass% or less, more preferably 2 mass% or less, and even more preferably 1 mass% or less. The content proportion of the compound represented by Formula (d) may be 0.1 mass% or greater.

The purity of the compound represented by Formula (1) in the epoxy compound product can be calculated as a ratio of peak areas obtained by gel permeation chromatography (GPC). When the shoulders of the peaks overlap, the peak areas are delimited by a line perpendicular to a base line, the perpendicular line passing through the valley between the peaks.

The content proportions of the compound represented by Formula (a), the compound represented by Formula (b), the compound represented by Formula (c), the compound represented by Formula (d), the compounds having a molecular weight of 100 or less, and the compounds having a molecular weight of 290 or greater can each be calculated as a ratio of the peak areas obtained by gas chromatography and mass spectrometry (GC-MS).

The Hazen color number (APHA) of the epoxy compound product is preferably 105 or less, more preferably 103 or less, even more preferably 100 or less, yet even more preferably 50 or less, and particularly preferably 15 or less.

From the perspective of achieving excellent handling properties, the viscosity at 25°C of the epoxy compound product is preferably 1300 mPa·s or less, more preferably 1200 mPa·s or less, even more preferably 1000 mPa·s or less, and particularly preferably 900 mPa·s or less. The viscosity is, for example, 50 mPa·s or greater, and may be 100 mPa·s or greater or 300 mPa·s or greater. Note that the viscosity is measured using a digital viscometer (Model No. "DVU-E II", available from Tokyo Keiki Inc.) under conditions including a standard rotor of 1°34' x R24, a temperature of 25°C, and a rotational speed of from 0.5 to 10 rpm.

Method for Producing Epoxy Compound Product

The epoxy compound product can be produced through the following epoxidation, the following low-boiling fraction removal, and the following high-boiling fraction removal. Note that the low-boiling fraction removal may be carried out before the high-boiling fraction removal, or the high-boiling fraction removal may be carried out before the low-boiling fraction removal.

Epoxidation: reacting 6-methyl-3-cyclohexenylmethyl(6'-methyl-3'-cyclohexenyl)carboxylate with an organic peracid to produce a reaction product
Low-boiling fraction removal: subjecting the reaction product to a low-boiling fraction removal treatment
High-boiling fraction removal: subjecting the reaction product to a high-boiling fraction removal treatment through thin film evaporation

In addition, the production method may include washing with water (water washing) the obtained reaction product to remove the organic peracid used in the reaction and the decomposition product thereof after completion of the epoxidation and before the low-boiling fraction removal (before the high-boiling fraction removal in a case in which the high-boiling fraction removal is carried out before the low-boiling fraction removal). Before the epoxidation, the production method may include subjecting crotonaldehyde and acrolein to a Diels-Alder reaction to obtain 6-methyl-1,3-cyclohexene-1-carboxaldehyde (subjecting to a Diels-Alder reaction) and/or subjecting 6-methyl-1,3-cyclohexene-1-carboxaldehyde to a Tischenko reaction to obtain 6-methyl-3-cyclohexenylmethyl(6'-methyl-3'-cyclohexenyl)carboxylate (subjecting to a Tischenko reaction).

(1) Epoxidation

The epoxidation is a process in which an organic peracid is reacted with 6-methyl-3-cyclohexenylmethyl(6'-methyl-3'-cyclohexenyl)carboxylate represented by Formula (A) below to produce a reaction product. Through this process, a reaction product containing a compound represented by Formula (1) is obtained.

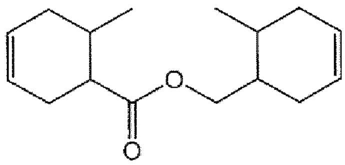

(A)

Examples of the organic peracid include performic acid, peracetic acid, perpropionic acid, m-chloroperbenzoic acid, trifluoroperacetic acid, and perbenzoic acid. A single type of the organic peracid may be used, or two or more types thereof may be used.

The usage amount of the organic peracid is, for example, from 0.5 to 3 mol per 1 mol of 6-methyl-3-cyclohexenylmethyl(6'-methyl-3'-cyclohexenyl)carboxylate.

The epoxidation reaction can be performed in the presence of a solvent. Examples of the solvent include aromatic hydrocarbons, such as benzene, toluene, xylene, ethylbenzene, isopropylbenzene, diethylbenzene, and p-cymene; alicyclic hydrocarbons, such as cyclohexane and decalin; aliphatic hydrocarbons, such as n-hexane, heptane, octane, nonane, and decane; alcohols, such as cyclohexanol, hexanol, heptanol, octanol, nonanol, and furfuryl alcohol; ketones, such as acetone, methyl ethyl ketone, and cyclohexanone; esters, such as ethyl acetate, n-amyl acetate, cyclohexyl acetate, isoamyl propionate, and methyl benzoate; polyhydric alcohols and their derivatives, such as ethylene glycol, propylene glycol, ethylene glycol monomethyl ether, ethylene glycol monomethyl ether acetate, ethylene glycol monoethyl ether, ethylene glycol monoethyl ether acetate, diethylene glycol monomethyl ether, and diethylene glycol monoethyl ether; halogen compounds, such as chloroform, dimethyl chloride, carbon tetrachloride, and chlorobenzene; and ethers, such as 1,2-dimethoxyethane. A single type of the solvent may be used, or two or more types thereof may be used.

The usage amount of the solvent is, for example, approximately from 0.2 to 10 times by mass the usage amount of 6-methyl-3-cyclohexenylmethyl(6'-methyl-3'-cyclohexenyl)carboxylate.

**In** the epoxidation reaction, a stabilizer for the organic peracid (such as, for example, ammonium hydrogen phosphate, potassium pyrophosphate, or 2-ethylhexyl tripolyphosphate), a polymerization inhibitor (such as, for example, hydroquinone, piperidine, ethanolamine, or phenothiazine), or the like may also be used as necessary.

The reaction temperature of the epoxidation reaction is, for example, from 0 to 70°C. The reaction atmosphere is not particularly limited and may be any atmosphere that does not inhibit the reaction, and may be, for example, any of an air atmosphere, a nitrogen atmosphere, and an argon atmosphere.

(2) Water Washing

The water washing is a process to remove, through washing with water, organic acids contained in the reaction product obtained through the epoxidation, the organic acids being the organic peracid and a decomposition product thereof.

The amount of water to be used is, for example, approximately from 0.1 to 3 times (v/v) that of the reaction product. An equilibrium extractor, such as a mixer-settler type equilibrium extractor, or an extraction column, a centrifugal extractor, or the like, can be used for the water washing.

(3) Low-Boiling Fraction Removal

The low-boiling fraction removal is a process to distill off a component (such as a solvent or moisture, for example) contained in the reaction product and having a boiling point lower than that of the compound represented by Formula (1). The reaction product is subjected to this process, and thereby the content of compounds having a molecular weight of 100 or less and mixed into the epoxy compound product can be reduced to a very low level.

In the low-boiling fraction removal, a thin film evaporator or distillation column can be used for the distillation. The distillation is preferably performed under conditions of a heating temperature ranging from 50 to 200°C and a pressure ranging from 1 to 760 torr. The distillation can also be carried out in two stages under different pressures and temperatures.

When the reaction product is subjected to the low-boiling fraction removal, a polymerization inhibitor is preferably added to suppress a ring-opening polymerization reaction of the compound represented by Formula (1). The addition amount of the polymerization inhibitor differs slightly depending on the type of the polymerization inhibitor and the distillation temperature and is preferably in a range of, for example, from 1 to 10000 ppm by mass (in particular, from 10 to

2000 ppm by mass) relative to the amount of the reaction product.

In the low-boiling fraction removal, a component with a boiling point lower than that of the compound represented by Formula (1) is evaporated and removed from the reaction product, and thereby a mixture of the compound represented by Formula (1) and a component with a boiling point higher than that of the compound represented by Formula (1) is obtained as a bottom liquid.

(4) High-boiling Fraction Removal

The high-boiling fraction removal is a process to distill off, through thin film evaporation, a component (such as a solvent or moisture, for example) contained in the reaction product and having a boiling point higher than that of the compound represented by Formula (1). When the high-boiling fraction removal is to be carried out after the low-boiling fraction removal, the high-boiling fraction removal is a process to evaporate and distill off the compound represented by Formula (1) from a mixture of the compound represented by Formula (1) and a component having a boiling point higher than that of the compound represented by Formula (1), the mixture being a bottom liquid obtained through the low-boiling fraction removal. By subjecting the mixture to this process, the content of compounds having a molecular weight of 290 or greater and mixed into the epoxy compound product can be reduced to a very low level, the compounds including the compounds represented by Formulas (a) to (d).

Preferably, the bottom liquid is introduced into a distillation column, the compound represented by Formula (1) is collected as a column top distillate, and a column bottom liquid containing a high-boiling point component is discharged out of the system.

Moreover, the treatment in the high-boiling fraction removal is preferably carried out under the above-described conditions because doing so results in a combination of the effects (1) to (4) below, and thereby the epoxy compound product containing the compound represented by Formula (1) with high purity is obtained.

(1) Suppressing production of the compound represented by Formula (a) as a byproduct by preventing an organic acid, which is a decomposition product of the organic peracid, from reacting with the compound represented by Formula (1);
(2) Suppressing a decrease in yield of the compound represented by Formula (1) by suppressing production of the compound represented by Formula (a) as a byproduct;
(3) Suppressing production of the compound represented by Formula (c) and the compound represented by Formula (d) as byproducts by preventing an organic acid, which is a decomposition product of the organic peracid, from reacting with the compound represented by Formula (a) produced as a byproduct; and
(4) Suppressing production of the compound represented by Formula (b) as a byproduct by preventing an organic acid, which is a decomposition product of the organic peracid, from reacting with 6-methyl-3-cyclohexenylmethyl(6'-methyl-3'-cyclohexenyl)carboxylate that remains unreacted.

As the distillation column, a packed column or a plate column, for example, can be used. The actual number of plates of the distillation column is, for example, not less than 14, and the actual number of plates is preferably from 14 to 100 and particularly preferably from 14 to 50 in terms of suppressing the inclusion of the compounds represented by Formulas (a) to (d) and further improving the purity of the product.

A thin film evaporator can be used for the distillation in the high-boiling fraction removal. The distillation is preferably carried out under conditions that include a heating temperature of 250°C or lower (preferably 230°C or lower) and a pressure of 3 torr or higher (preferably 0.7 torr or lower) from the perspective of suppressing decomposition of the compound represented by Formula (1), which increases the degree of coloration, and suppressing ring-opening polymerization of the epoxy group of the compound represented by Formula (1), which leads to formation of a gel. The distillation temperature is preferably 170°C or higher, and more preferably 180°C of higher. From the viewpoint of being able to further increase the purity of the epoxy compound product, the pressure is preferably 0.01 torr or greater, and may be 0.02 torr or greater. Moreover, from the viewpoint of being able to further suppress the production of the compounds represented by Formulae (a) to (d) as byproducts, the pressure is preferably 0.5 Torr or less, more preferably 0.2 Torr or less, even more preferably 0.16 Torr or less, yet even more preferably 0.1 Torr or less, and particularly preferably 0.04 Torr or less.

The wiper rotational speed is preferably from 100 to 800 rpm, and more preferably from 200 to 600 rpm. When the wiper rotational speed is too high, the energy costs tend to increase, and conversely, when the wiper rotational speed is too low, the compounds represented by Formulae (a) to (d) tend to be readily incorporated in the product.

It has hitherto been difficult to produce the epoxy compound product by repeating a simple distillation. This is because in distillation to separate from high-boiling point components particularly, the compound represented by Formula (1) or 6-methyl-3-cyclohexenylmethyl(6'-methyl-3'-cyclohexenyl)carboxylate, which is a precursor of the compound represented by Formula (1), reacts through heating with an organic acid that is a decomposition product of the organic

peracid, and thereby the compounds represented by Formulae (a) to (d) are reproduced. Furthermore, the compound represented by Formula (1) has a higher boiling point than 3,4-epoxycyclohexylmethyl(3',4'-epoxy)cyclohexane carboxylate, and thus the distillation needs to be carried out at a relatively high temperature. Therefore, in the high-boiling fraction removal, the reactions that produce the compounds represented by Formulae (a) to (d) more easily advance, and as a result, the purity of the obtained epoxy compound product is easily reduced. However, by carrying out thin film distillation in the high-boiling fraction removal, the compound represented by Formula (1) can be efficiently volatilized without excessively increasing the heating temperature, and therefore, the reactions that produce the compounds represented by Formulae (a) to (d) can be suppressed in the high-boiling fraction removal, and a high-purity epoxy compound product can be obtained. Moreover, by controlling the pressure in the high-boiling fraction removal, an epoxy compound product of higher purity can be obtained.

Curable Composition

The compound represented by Formula (1) is a curable compound, and a curable composition can be obtained by using the epoxy compound product. The curable composition contains the epoxy compound product described above.

Curable Compound

The curable composition contains, as a curable compound, at least the compound represented by Formula (1) and included in the epoxy compound product described above. The curable composition may include another curable compound in addition to the compound represented by Formula (1). The curable composition may include a single type of other curable compound, or may include two or more types of other curable compounds.
Examples of the other curable compound include other epoxy compounds besides the compound represented by Formula (1), a compound having one or more oxetane groups in the molecule (such a compound may be referred to as an "oxetane compound"), and a compound having one or more vinyl ether groups in the molecule (such a compound may be referred to as a "vinyl ether compound"). The curable composition may contain, as the other compound described above, another epoxy compound and/or oxetane compound described above.
Examples of the other epoxy compound include compounds having one or more epoxy groups (oxiranyl group) in the molecule. Among these, the other epoxy compound is preferably a compound having two or more (preferably from 2 to 6, more preferably from 2 to 4) epoxy groups in the molecule.
Examples of the other epoxy compound include an alicyclic epoxy compound, an aromatic epoxy compound, and an aliphatic epoxy compound.
Examples of the alicyclic epoxy compound include well-known or commonly used compounds that have one or more alicyclic rings and one or more epoxy groups in the molecule. Such an alicyclic epoxy compound is not particularly limited, and examples thereof include (I) a compound having an epoxy group (referred to as an "alicyclic epoxy group") composed of an oxygen atom and two adjacent carbon atoms constituting an alicyclic ring in the molecule; (II) a compound in which an epoxy group is directly bonded to an alicyclic ring via a single bond, and (III) a compound having an alicyclic ring and a glycidyl ether group in the molecule (a glycidyl ether type epoxy compound).
Examples of the compound (I) having an alicyclic epoxy group in the molecule include compounds represented by Formula (i) below.

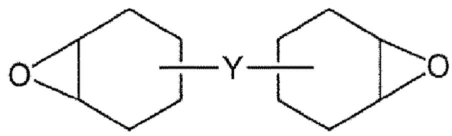 (i)

In Formula (i), Y represents a single bond or a linking group (a divalent group having one or more atoms). Examples of the linking group include a divalent hydrocarbon group, an alkenylene group in which some or all of the carbon-carbon double bonds are epoxidized, a carbonyl group, an ether bond, an ester bond, a carbonate group, an amide group, and a linked group in which a plurality of the above groups are linked. Note that substituents such as alkyl groups may be bonded to one or more of the carbon atoms constituting the cyclohexane ring (cyclohexene oxide group) in Formula (i).
Examples of the divalent hydrocarbon group include a linear or branched alkylene group having from 1 to 18 carbons and a divalent alicyclic hydrocarbon group. Examples of the linear or branched alkylene group having from 1 to 18 carbons include a methylene group, a methylmethylene group, a dimethylmethylene group, an ethylene group, a propylene group, and a trimethylene group. Examples of the divalent alicyclic hydrocarbon group include divalent cycloalkylene groups (including cycloalkylidene groups), such as a 1,2-cyclopentylene group, a 1,3-cyclopentylene group, a cyclopentylidene group, a 1,2-cyclohexylene group, a 1,3-cyclohexylene group, a 1,4-cyclohexylene group, and

a cyclohexylidene group.

In the alkenylene group in which some or all of the carbon-carbon double bonds are epoxidized (the alkenylene group may be referred to as an "epoxidized alkenylene group"), examples of the alkenylene group include linear or branched alkenylene groups having from 2 to 8 carbons, such as a vinylene group, a propenylene group, a 1-butenylene group, a 2-butenylene group, a butadienylene group, a pentenylene group, a hexenylene group, a heptenylene group, and an octenylene group. In particular, the epoxidized alkenylene group is preferably an epoxidized alkenylene group in which all of the carbon-carbon double bonds are epoxidized, and more preferably an epoxidized alkenylene group having from 2 to 4 carbons in which all of the carbon-carbon double bonds are epoxidized.

Representative examples of the alicyclic epoxy compound represented by Formula (i) include (3,4,3',4'-diepoxy) bicyclohexyl and compounds represented by Formulae (i-1) to (i-9) below. In Formulae (i-4) and (i-6) below, l and m each represent an integer from 1 to 30. R' in Formula (i-4) below is an alkylene group having from 1 to 8 carbons, and, among these, a linear or branched alkylene group having from 1 to 3 carbons, such as a methylene group, an ethylene group, a propylene group, or an isopropylene group, is preferable. In Formulae (i-8) and (i-9) below, n1 to n6 each represent an integer from 1 to 30. In addition, other examples of the alicyclic epoxy compound represented by the formula (i) include 2,2-bis(3,4-epoxycyclohexyl)propane, 1,2-bis(3,4-epoxycyclohexan-1-yl)ethane, 1,2-epoxy-1,2-bis(3,4-epoxycyclohexan-1-yl)ethane, and bis(3,4-epoxycyclohexylmethyl)ether.

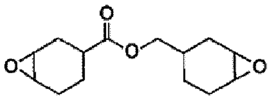

(i-1)

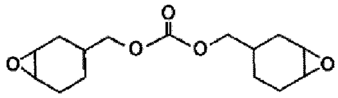

(i-2)

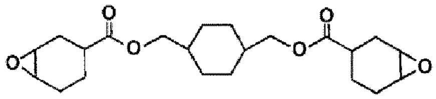

(i-3)

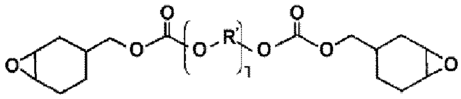

(i-4)

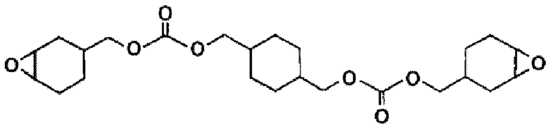

(i-5)

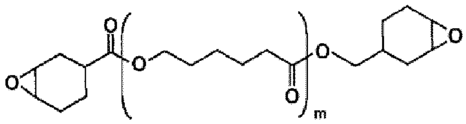

(i-6)

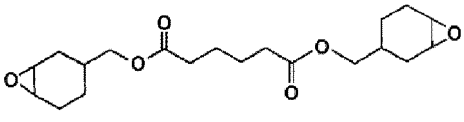

(i-7)

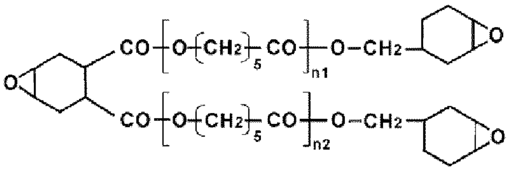

(i-8)

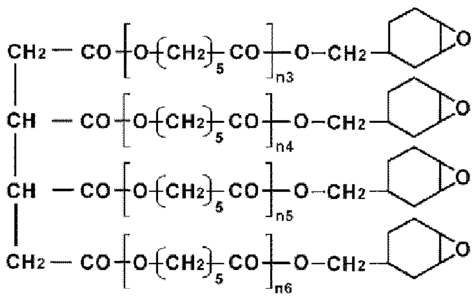

(i−9)

Moreover, examples of the compound (I) having an alicyclic epoxy group in the molecule include epoxy-modified siloxanes. Examples of the epoxy-modified siloxanes include a chain or cyclic polyorganosiloxane having a constituent unit represented by Formula (i') below.

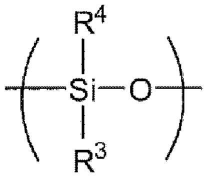

(i ')

In Formula (i'), $R^3$ represents a substituent containing a group represented by Formula (1a) below or a substituent containing a group represented by Formula (1b) below, and $R^4$ represents an alkyl group or an alkoxy group.

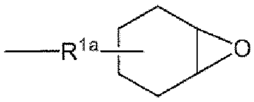

(1a)

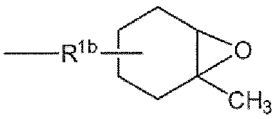

(1b)

In Formula (1a) and Formula (1b), $R^{1a}$ and $R^{1b}$ may be the same or different and each represent a linear or branched alkylene group, and examples thereof include linear or branched alkylene groups having from 1 to 10 carbons, such as a methylene group, a methylmethylene group, a dimethylmethylene group, an ethylene group, a propylene group, a trimethylene group, a tetramethylene group, a pentamethylene group, a hexamethylene group, and a decamethylene group.

The epoxy equivalent (in accordance with JIS K7236) of the epoxy-modified siloxane is, for example, from 100 to 400 and preferably from 150 to 300.

As the epoxy-modified siloxane, for example, a commercially available product can be used, such as, for example, a compound represented by Formula (i'-1) below (trade name "KR-470", available from Shin-Etsu Chemical Co., Ltd.).

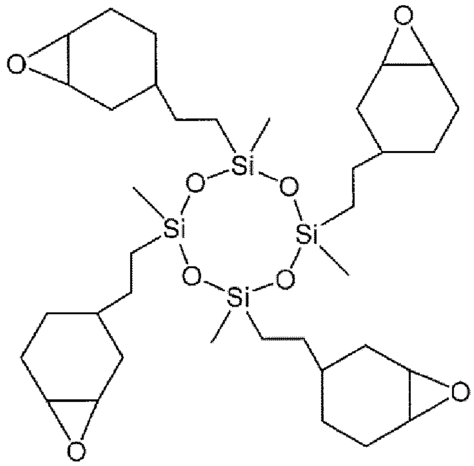

(i'−1)

Examples of the compound (II) in which an epoxy group is directly bonded to an alicyclic ring via a single bond include compounds represented by Formula (ii) below.

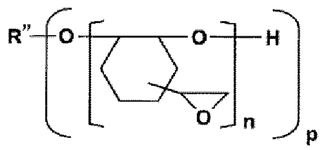

(ii)

In Formula (ii), R" is a group (p-valent organic group) resulting from elimination of a quantity of p hydroxyl groups (-OH) from a structural formula of a p-hydric alcohol, wherein p and n each represent a natural number. Examples of the p-hydric alcohol [R"(OH)p] include polyhydric alcohols (such as alcohols having from 1 to 15 carbons), such as 2,2-bis(hydroxymethyl)-1-butanol. Here, p is preferably from 1 to 6, and n is preferably from 1 to 30. When p is 2 or greater, n in each group within the brackets [] (within the outer brackets) may be the same or different. Examples of the compound represented by Formula (ii) specifically include a 1,2-epoxy-4-(2-oxiranyl)cyclohexane adduct of 2,2-bis(hydroxymethyl)-1-butanol (such as, for example, a product of the trade name "EHPE3150" (available from Daicel Corporation)).

Examples of the compound (III) having an alicyclic ring and a glycidyl ether group in the molecule include glycidyl ethers of alicyclic alcohols (in particular, alicyclic polyhydric alcohols). More particularly, examples of the compound (III) include compounds obtained by hydrogenating a bisphenol A type epoxy compound (a hydrogenated bisphenol A type epoxy compound), such as 2,2-bis[4-(2,3-epoxypropoxy)cyclohexyl]propane and 2,2-bis[3,5-dimethyl-4-(2,3-epoxypropoxy)cyclohexyl]propane; compounds obtained by hydrogenating a bisphenol F type epoxy compound (a hydrogenated bisphenol F type epoxy compound), such as bis[o,o-(2,3-epoxypropoxy)cyclohexyl]methane, bis[o,p-(2,3-epoxypropoxy)cyclohexyl]methane, bis[p,p-(2,3-epoxypropoxy)cyclohexyl]methane, and bis[3,5-dimethyl-4-(2,3-epoxypropoxy)cyclohexyl]methane; hydrogenated bisphenol type epoxy compounds; hydrogenated phenol novolac type epoxy compounds; hydrogenated cresol novolac type epoxy compounds; hydrogenated cresol novolac type epoxy compounds of bisphenol A; hydrogenated naphthalene type epoxy compounds; hydrogenated epoxy compounds of an epoxy compound obtained from trisphenolmethane; and other hydrogenated epoxy compounds of an epoxy compound having an aromatic ring.

The aromatic epoxy compound is a compound having one or more aromatic rings (aromatic hydrocarbon rings or aromatic heterocycles) and one or more epoxy groups in the molecule. As the aromatic epoxy compound, a compound (aromatic glycidyl ether-based epoxy compound) in which a glycidoxy group is bonded to one or more carbon atoms constituting a carbon atom-containing aromatic ring (particularly, an aromatic hydrocarbon ring) is preferable.

Examples of the aromatic epoxy compound include an epi-bis type glycidyl ether type epoxy resins obtained by a condensation reaction of a bisphenol (such as, for example, bisphenol A, bisphenol F, bisphenol S, or fluorenebisphenol) and an epihalohydrin; a high molecular weight epi-bis type glycidyl ether type epoxy resin obtained by further subjecting the above epi-bis type glycidyl ether type epoxy resin to an addition reaction with the above bisphenol; a novolac alkyl type glycidyl ether type epoxy resin obtained by subjecting a phenol (such as, for example, phenol, cresol, xylenol, resorcin, catechol, bisphenol A, bisphenol F, or bisphenol S) and an aldehyde (such as, for example, formaldehyde, acetaldehyde, benzaldehyde, hydroxybenzaldehyde, or salicylaldehyde) to a condensation reaction to obtain a polyhydric alcohol, and then further subjecting the polyhydric alcohol to a condensation reaction with epihalohydrin; and an epoxy compound in which two phenolic backbones are bonded to the 9-position of the fluorene ring, and glycidyl groups are each bonded directly or via an alkyleneoxy group to an oxygen atom obtained by removing a hydrogen atom from the hydroxy group of these phenolic backbones.

Examples of the aliphatic epoxy compound include a glycidyl ether of a q-hydric alcohol having no cyclic structure (q is a natural number); a glycidyl ester of a monovalent or polyvalent carboxylic acid (such as, for example, acetic acid, propionic acid, butyric acid, stearic acid, adipic acid, sebacic acid, maleic acid, and itaconic acid); an epoxidized product of an oil and/or fat having a double bond, such as an epoxidized linseed oil, an epoxidized soybean oil, and an epoxidized castor oil; and an epoxidized product of a polyolefin (including a polyalkadiene), such as an epoxidized polybutadiene. Note that examples of the q-hydric alcohol having no cyclic structure include a monohydric alcohol, such as methanol, ethanol, 1-propyl alcohol, isopropyl alcohol, and 1-butanol; a dihydric alcohol, such as ethylene glycol, 1,2-propanediol, 1,3-propanediol, 1,4-butanediol, neopentyl glycol, 1,6-hexanediol, diethylene glycol, triethylene glycol, tetraethylene glycol, dipropylene glycol, polyethylene glycol, and polypropylene glycol; and a trihydric or higher polyhydric alcohol, such as glycerol, diglycerol, erythritol, trimethylolethane, trimethylolpropane, pentaerythritol, dipentaerythritol, and sorbitol. **In** addition, the q-hydric alcohol may be a polyether polyol, a polyester polyol, a polycarbonate polyol, a polyolefin polyol, or the like.

The oxetane compound is not particularly limited and includes well-known or commonly used compounds including one or more oxetane rings in the molecule. Examples thereof include 3,3-bis(vinyloxymethyl)oxetane, 3-ethyl-3-(hydroxymethyl)oxetane, 3-ethyl-3-(2-ethylhexyloxymethyl)oxetane, 3-ethyl-3-[(phenoxy)methyl]oxetane, 3-ethyl-3-(hexyloxymethyl)oxetane, 3-ethyl-3-(chloromethyl)oxetane, 3,3-bis(chloromethyl)oxetane, 1,4-bis[(3-ethyl-3-oxetanylmethoxy)methyl]benzene, bis{[1-ethyl(3-oxetanyl)]methyl}ether, 4,4'-bis[(3-ethyl-3-oxetanyl)methoxymethyl]bicyclohexyl, 1,4-bis[(3-ethyl-3-oxetanyl)methoxymethyl]cyclohexane, 1,4-bis{[(3-ethyl-3-oxetanyl)methoxy]methyl}benzene, 3-ethyl-3-{[(3-ethyloxetane-3-yl)methoxy]methyl}oxetane, xylylenebisoxetane, 3-ethyl-3-{[3-(triethoxysilyl)propoxy]methyl}oxetane, oxetanylsilsesquioxane, and phenol novolac oxetane.

The vinyl ether compound is not particularly limited, and a well-known or commonly used compound including one or more vinyl ether groups in the molecule can be used. Examples thereof include 2-hydroxyethyl vinyl ether (ethylene glycol monovinyl ether), 3-hydroxypropyl vinyl ether, 2-hydroxypropyl vinyl ether, 2-hydroxyisopropyl vinyl ether, 4-hydroxybutyl vinyl ether, 3-hydroxybutyl vinyl ether, 2-hydroxybutyl vinyl ether, 3-hydroxyisobutyl vinyl ether, 2-hydroxyisobutyl vinyl ether, 1-methyl-3-hydroxypropyl vinyl ether, 1-methyl-2-hydroxypropyl vinyl ether, 1-hydroxymethylpropyl vinyl ether, 4-hydroxycyclohexyl vinyl ether, 1,6-hexanediol monovinyl ether, 1,6-hexanediol divinyl ether, 1,8-octanediol divinyl ether, 1,4-cyclohexanedimethanol monovinyl ether, 1,4-cyclohexanedimethanol divinyl ether, 1,3-cyclohexanedimethanol monovinyl ether, 1,3-cyclohexanedimethanol divinyl ether, 1,2-cyclohexanedimethanol monovinyl ether, 1,2-cyclohexanedimethanol divinyl ether, p-xylene glycol monovinyl ether, p-xylene glycol divinyl ether, m-xylene glycol monovinyl ether, m-xylene glycol divinyl ether, o-xylene glycol monovinyl ether, o-xylene glycol divinyl ether, ethylene glycol divinyl ether, diethylene glycol monovinyl ether, diethylene glycol divinyl ether, triethylene glycol monovinyl ether, triethylene glycol divinyl ether, tetraethylene glycol monovinyl ether, tetraethylene glycol divinyl ether, pentaethylene glycol monovinyl ether, pentaethylene glycol divinyl ether, oligoethylene glycol monovinyl ether, oligoethylene glycol divinyl ether, polyethylene glycol monovinyl ether, polyethylene glycol divinyl ether, dipropylene glycol monovinyl ether, dipropylene glycol divinyl ether, tripropylene glycol monovinyl ether, tripropylene glycol divinyl ether, tetrapropylene glycol monovinyl ether, tetrapropylene glycol divinyl ether, pentapropylene glycol monovinyl ether, pentapropylene glycol divinyl ether, oligopropylene glycol monovinyl ether, oligopropylene glycol divinyl ether, polypropylene glycol monovinyl ether, polypropylene glycol divinyl ether, isosorbide divinyl ether, oxanorbornene divinyl ether, phenyl vinyl ether, n-butyl vinyl ether, isobutyl vinyl ether, octyl vinyl ether, cyclohexyl vinyl ether, hydroquinone divinyl ether, 1,4-butanediol divinyl ether, cyclohexanedimethanol divinyl ether, trimethylolpropane divinyl ether, trimethylolpropane trivinyl ether, bisphenol A divinyl ether, bisphenol F divinyl ether, hydroxyoxanorbornane methanol divinyl ether, 1,4-cyclohexanediol divinyl ether, pentaerythritol trivinyl ether, pentaerythritol tetravinyl ether, dipentaerythritol pentavinyl ether, and dipentaerythritol hexavinyl ether.

A proportion of the compound represented by Formula (1) in a total amount (100 mass%) of the curable compound contained in the curable composition is, for example, 50 mass% or greater (for example, from 50 to 100 mass%), preferably 60 mass% or greater, particularly preferably 70 mass% or greater, and most preferably 80 mass% or greater.

The curable composition preferably contains, in addition to the curable compound, for example, one or more selected from the group consisting of a curing agent, a curing accelerator, and a curing catalyst. The curable composition preferably contains a curing agent and/or a curing catalyst.

The content proportion of a total of the curable compound and the curing agent and/or the curing accelerator in the total amount (100 mass%) of the curable composition is, for example, 60 mass% or greater, preferably 70 mass% or greater, more preferably 80 mass% or greater, even more preferably 90 mass% or greater, and particularly preferably 95 mass% or greater.

In addition, the content proportion of the total of the curable compound and the curing catalyst in the total amount (100 mass%) of the curable composition is, for example, 60 mass% or greater, preferably 70 mass% or greater, more preferably 80 mass% or greater, even more preferably 90 mass% or greater, and particularly preferably 95 mass% or greater.

The content proportion of compounds other than the curable compound, the curing agent, the curing accelerator, and the curing catalyst relative to the total amount (100 mass%) of the curable composition is, for example, 50 mass% or less, and preferably 40 mass% or less.

Curing agent

Examples of the curing agent that can be used include curing agents that are well-known or commonly used as curing agents for epoxy resins, such as acid anhydrides (acid anhydride-based curing agents), amines (amine-based curing agents), polyamide resins, imidazoles (imidazole-based curing agents), polymercaptans (polymercaptan-based curing agents), phenols (phenol-based curing agents), polycarboxylic acids, dicyandiamides, and organic acid hydrazides. A single type of the curing agent may be used alone, or two or more types thereof may be used.

Examples of the acid anhydrides include methyltetrahydrophthalic anhydrides (such as 4-methyltetrahydrophthalic anhydride and 3-methyltetrahydrophthalic anhydride), methylhexahydrophthalic anhydride (such as 4-methylhexahydrophthalic anhydride and 3-methylhexahydrophthalic anhydride), dodecenyl succinic anhydride, methyl endomethylene tetrahydrophthalic anhydride, phthalic anhydride, maleic anhydride, tetrahydrophthalic anhydride, hexahydrophthalic anhydride, methylcyclohexene dicarboxylic anhydride, pyromellitic anhydride, trimellitic anhydride, benzophenone tetracarboxylic anhydride, nadic anhydride, methyl nadic anhydride, hydrogenated methyl nadic anhydride, 4-(4-methyl-3-pentenyl)tetrahydrophthalic anhydride, succinic anhydride, adipic anhydride, sebacic anhydride, dodecanedioic anhydride, methylcyclohexene tetracarboxylic anhydride, vinyl ether-maleic anhydride copolymers, and alkyl styrene-maleic anhydride copolymers. Among these, from the viewpoint of handling properties, acid anhydrides that are liquid at 25°C (such as, for example, methyltetrahydrophthalic anhydride, methylhexahydrophthalic anhydride, dodecenyl succinic anhydride, and methyl endomethylene tetrahydrophthalic anhydride) are preferred.

Examples of the amines include aliphatic polyamines, such as ethylenediamine, diethylenetriamine, triethylenetetramine, tetraethylenepentamine, dipropylenediamine, diethylaminopropylamine, and polypropylenetriamine; alicyclic polyamines, such as menthenediamine, isophoronediamine, bis(4-amino-3-methyldicyclohexyl)methane, diaminodicyclohexylmethane, bis(aminomethyl)cyclohexane, N-aminoethyl piperazine, and 3,9-bis(3-aminopropyl)-3,4,8,10-tetraoxaspiro[5,5]undecane; mononuclear polyamines, such as m-phenylenediamine, p-phenylenediamine, tolylene-2,4-diamine, tolylene-2,6-diamine, mesitylene-2,4-diamine, 3,5-diethyltolylene-2,4-diamine, and 3,5-diethyltolylene-2,6-diamine; and aromatic polyamines, such as biphenylenediamine, 4,4-diaminodiphenylmethane, 2,5-naphthylenediamine, and 2,6-naphthylenediamine.

Examples of the polyamide resins include polyamide resins having a primary amino group and/or a secondary amino group in the molecule.

Examples of the imidazoles include 2-methylimidazole, 2-ethyl-4-methylimidazole, 2-undecylimidazole, 2-heptadecylimidazole, 2-phenylimidazole, 1-benzyl-2-methylimidazole, 1-cyanoethyl-2-methylimidazole, 1-cyanoethyl-2-ethyl-4-methylimidazole, 1-cyanoethyl-2-undecylimidazole, 1-cyanoethyl-2-undecylimidazolium trimellitate, 1-cyanoethyl-2-phenylimidazolium trimellitate, 2-methylimidazolium isocyanurate, 2-phenylimidazolium isocyanurate, 2,4-diamino-6-[2-methylimidazolyl-(1)]-ethyl-s-triazine, and 2,4-diamino-6-[2-ethyl-4-methylimidazolyl-(1)]-ethyl-s-triazine.

Examples of the polymercaptans include liquid polymercaptans and polysulfide resins.

Examples of the phenols include aralkyl resins, such as novolac phenolic resins, novolac cresol resins, p-xylylene-modified phenolic resins, and p-xylylene/m-xylylene-modified phenolic resins; terpene-modified phenolic resins; dicyclopentadiene-modified phenolic resins, and triphenolpropane.

Examples of the polycarboxylic acids include adipic acid, sebacic acid, terephthalic acid, trimellitic acid, and carboxy group-containing polyesters.

Among the curing agents, the curing agent is preferably an acid anhydride (acid anhydride curing agent) from the viewpoints of heat resistance and transparency of the resulting cured product, and a commercially available product can be used, such as, for example, "RIKACID MH-700 (trade name)" and "RIKACID MH-700F (trade name)" (both available from New Japan Chemical Co., Ltd.), and "HN-5500 (trade name)" (available from Hitachi Chemical Co., Ltd.).

The content (amount to be blended) of the curing agent is preferably from 50 to 200 parts by mass and more preferably from 80 to 150 parts by mass relative to 100 parts by mass of a total amount of epoxy compounds contained in the curable composition. More specifically, when an acid anhydride is used as a curing agent, the acid anhydride is preferably used at a proportion of from 0.5 to 1.5 equivalents per equivalent of epoxy groups in all the epoxy compounds included in the curable composition. When the content of the curing agent is 50 parts by mass or greater, the curing reaction can proceed sufficiently, and the toughness of the resulting cured product tends to improve. Meanwhile, when the content of the curing agent is 200 parts by mass or less, discoloration is further suppressed, and the obtained cured product tends to exhibit an excellent hue.

Curing accelerator

When the curable composition contains a curing agent, the curable composition preferably further contains a curing accelerator. The curing accelerator has an effect of accelerating the reaction rate when a compound having an epoxy group (oxiranyl group) reacts with the curing agent.

Examples of the curing accelerator include 1,8-diazabicyclo [5.4.0] undecene-7 (DBU) or salts thereof (such as, for example, a phenol salt, an octylate salt, a p-toluene sulfonate salt, a formate salt, and a tetraphenylborate salt); 1,5-diazabicyclo[4.3.0]nonene-5 (DBN) or salts thereof (such as, for example, a phenol salt, an octylate salt, a p-toluene sulfonate salt, a formate salt, and a tetraphenylborate salt); tertiary amines, such as benzyldimethylamine, 2,4,6-tris(dimethylaminomethyl) phenol, and N,N-dimethylcyclohexylamine; imidazoles, such as 2-ethyl-4-methylimidazole and 1-cyanoethyl-2-ethyl-4-methylimidazole; phosphoric esters; phosphines, such as triphenylphosphine and tris(dimethoxy)phosphine; phosphonium compounds, such as tetraphenylphosphonium tetra(p-tolyl)borate; organometallic salts, such as zinc octylate, tin octylate, and zinc stearate; and metal chelates, such as aluminum acetylacetone complex. A single type of the curing accelerator may be used alone, or two or more types thereof may be used.

As the curing accelerator, a commercially available product can be used, such as, for example, products of the trade names "U-CAT SA 506", "U-CAT SA 102", "U-CAT 5003", "U-CAT 18X ", and "U-CAT 12XD" (under development) (the above products being available from San-Apro Ltd.); products of the trade names "TPP-K" and "TPP-MK" (the above products being available from Hokko Chemical Industry Co., Ltd.); and a product of the trade name "PX-4ET" (available from Nippon Chemical Industrial Co., Ltd.).

The content (amount to be blended) of the curing accelerator is preferably from 0.01 to 5 parts by mass, more preferably from 0.02 to 3 parts by mass, and even more preferably from 0.03 to 3 parts by mass relative to 100 parts by mass of the curing agent. When the content of the curing accelerator is 0.01 parts by mass or greater, the curing accelerator tends to provide a more efficient curing acceleration effect. Meanwhile, when the content of the curing accelerator is 5 parts by mass or less, discoloration is suppressed, and the obtained cured product tends to exhibit an excellent hue.

Curing Catalyst

The curable composition may contain a curing catalyst in place of the curing agent. The curing catalyst has a function of curing the curable composition by initiating and/or accelerating the curing reaction (polymerization reaction) of a cationic curable compound, such as the compound represented by Formula (1). Examples of the curing catalyst include cationic polymerization initiators (such as photocationic polymerization initiators and thermal cationic polymerization initiators), which initiate polymerization by generating cationic species upon, for example, light irradiation or a heating treatment; Lewis acid-amine complexes; Bronsted acid salts; and imidazoles. A single type of the curing catalyst may be used alone, or two or more types thereof may be used.

Examples of the photocationic polymerization initiators include hexafluoroantimonate salts, pentafluorohydroxyantimonate salts, hexafluorophosphate salts, and hexafluoroarsenate salts, and more specifically include sulfonium salts (in particular, triarylsulfonium salts), such as triarylsulfonium hexafluorophosphate (such as, for example, p-phenylthiophenyl diphenylsulfonium hexafluorophosphate) and triarylsulfonium hexafluoroantimonate; iodonium salts, such as diaryliodonium hexafluorophosphate, diaryliodonium hexafluoroantimonate, bis(dodecylphenyl)iodonium tetrakis(pentafluorophenyl)borate, and iodonium [4-(4-methylphenyl-2-methylpropyl)phenyl]hexafluorophosphate; phosphonium salts, such as tetrafluorophosphonium hexafluorophosphate; and pyridinium salts, such as N-hexylpyridinium tetrafluoroborate. In addition, as the photocationic polymerization initiator, a commercially available product can be preferably used, such as, for example, a product of the trade name "UVACURE 1590" (available from Daicel-Allnex Ltd.); products of the trade names "CD-1010", "CD-1011", and "CD-1012" (the above products being available from Sartomer USA, LLC); a product of the trade name "Irgacure 264" (available from BASF SE); and a product of the trade name "CIT-1682" (available from Nippon Soda Co., Ltd.).

Examples of the cationic polymerization initiators include aryldiazonium salts, aryliodonium salts, arylsulfonium salts, and allene-ion complexes. A commercially available product can be preferably used, such as, for example, products of the trade names "PP-33", "CP-66", and "CP-77" (the above products being available from Adeka Corporation); a product of the trade name "FC-509" (available from 3M Company); a product of the trade name "UVE1014" (available from G.E.); products of the trade names "SAN-AID SI-60L", "SAN-AID SI-80L", "SAN-AID SI-100L", "SAN-AID SI-110L", and "SAN-AID SI-150L" (the above products being available from Sanshin Chemical Industry Co., Ltd.); and a product of the trade name "CG-24-61)" (available from BASF SE).

Examples of the Lewis acid-amine complexes include $BF_3$•n-hexylamine, $BF_3$•monoethylamine, $BF_3$•benzylamine, $BF_3$•diethylamine, $BF_3$•piperidine, $BF_3$•triethylamine, $BF_3$•aniline, $BF_4$•n-hexylamine, $BF_4$•monoethylamine, $BF_4$•benzylamine, $BF_4$•diethylamine, $BF_4$•piperidine, $BF_4$•triethylamine, $BF_4$•aniline, $PF_5$•ethylamine, $PF_5$•isopropylamine, $PF_5$•butylamine, $PF_5$•laurylamine, $PF_5$•benzylamine, and $AsF_5$•laurylamine.

Examples of the Brønsted acid salt include aliphatic sulfonium salts, aromatic sulfonium salts, iodonium salts,

and phosphonium salts.

Examples of the imidazoles include 2-methylimidazole, 2-ethyl-4-methylimidazole, 2-undecylimidazole, 2-heptadecylimidazole, 2-phenylimidazole, 1-benzyl-2-methylimidazole, 1-cyanoethyl-2-methylimidazole, 1-cyanoethyl-2-ethyl-4-methylimidazole, 1-cyanoethyl-2-undecylimidazole, 1-cyanoethyl-2-undecylimidazolium trimellitate, 1-cyanoethyl-2-phenylimidazolium trimellitate, 2-methylimidazolium isocyanurate, 2-phenylimidazolium isocyanurate, 2,4-diamino-6-[2-methylimidazolyl-(1)]-ethyl-s-triazine, and 2,4-diamino-6-[2-ethyl-4-methylimidazolyl-(1)]-ethyl-s-triazine.

The content (amount to be blended) of the curing catalyst is preferably from 0.01 to 5 parts by mass, more preferably from 0.02 to 4 parts by mass, and even more preferably from 0.03 to 3 parts by mass relative to 100 parts by mass of the cationic curable compound contained in the curable composition. When the content of the curing catalyst is within the above range, the curing rate of the curable composition increases, and the heat resistance and transparency of the cured product tend to improve in a well-balanced manner.

The curable composition may contain an additive as necessary in addition to the above-described components. Examples of the additive include polyhydric alcohols, such as ethylene glycol, diethylene glycol, propylene glycol, and glycerin; antifoaming agents, leveling agents, silane coupling agents, surfactants, inorganic fillers, flame retardants, colorants, ion adsorbents, pigments, fluorescents, and release agents. A single type of the additives may be used alone, or two or more types of the additives may be used.

The curable composition can be prepared by stirring and mixing each component described above in a heated state as necessary. For the stirring and mixing, a well-known or commonly used stirring and mixing means can be used, such as, for example, a mixer of various types, such as a dissolver and a homogenizer; a kneader; a roll mill; a bead mill; and a rotating/revolving-type stirring apparatus. In addition, after the stirring and mixing, the mixture may be defoamed under vacuum.

In the curable composition, the proportion of the compound represented by Formula (1) relative to the total amount (100 mass%) of the compound represented by Formula (1), the compound represented by Formula (a), the compound represented by Formula (b), the compound represented by Formula (c), and the compound represented by Formula (d) is 90 mass% or greater, preferably 91 mass% or greater, more preferably 94 mass% or greater, and still more preferably 96 mass% or greater. This proportion can be calculated from a ratio of peak areas obtained by GC-MS.

In the curable composition, the total proportion of the compound represented by Formula (a), the compound represented by Formula (b), the compound represented by Formula (c), and the compound represented by Formula (d) relative to the total amount (100 mass%) of the compound represented by Formula (1), the compound represented by Formula (a), the compound represented by Formula (b), the compound represented by Formula (c), and the compound represented by Formula (d) is 10 mass% or less, preferably 9 mass% or less, more preferably 6 mass% or less, and still more preferably 4 mass% or less. This proportion can be calculated from a ratio of peak areas obtained by GC-MS.

The curable composition has a fast curing property, and the curing time (or gelling time) at 120°C is, for example, 1100 seconds or less and preferably 1050 seconds or less. The curing time (or gelling time) at 80°C of the curable composition is, for example, 5000 seconds or less, preferably 4000 seconds or less, and more preferably 3000 seconds or less.

The heating temperature (curing temperature) during curing is preferably from 45 to 200°C, more preferably from 100 to 190°C, and even more preferably from 100 to 180°C. In addition, the heating time (or curing time) is preferably from 30 to 600 minutes and more preferably from 45 to 540 minutes. A heating temperature or heating time that is less than the range described above results in insufficient curing, and conversely, a heating temperature or heating time that exceeds the above range may cause decomposition of the resin component. Thus, both such deviations are not preferred. Although the curing conditions depend on various conditions, the curing conditions can be appropriately adjusted, for example, by shortening the heating time when the heating temperature is increased, or increasing the heating time when the heating temperature is reduced.

Cured product

A cured product is obtained by curing the curable composition described above. The cured product is excellent in transparency and heat resistance.

The cured product is excellent in transparency, and the light transmittance (at a thickness of 3 mm) thereof for light at a wavelength of 450 nm is, for example, preferably 80% or greater, more preferably 85% or greater, even more preferably 88% or greater, and particularly preferably 90% or greater. The curable composition forms a cured product excellent in transparency and thus, when the curable composition is used as an encapsulant, a die attachment paste agent, or the like of an optical semiconductor element in an optical semiconductor device, the light intensity emitted from the optical semiconductor device tends to further increase.

The cured product exhibits excellent heat resistance, and the glass transition temperature (Tg) thereof is preferably 170°C or higher, more preferably 175°C or higher, still more preferably 180°C or higher, yet even more

preferably 190°C or higher, and particularly preferably 200°C or higher.

The cured product is excellent in heat resistance, and the 5% weight loss temperature (Td5) is preferably 325°C or higher, more preferably 330°C or higher, and still more preferably 335°C or higher. The 10% weight loss temperature (Td10) of the cured product is preferably 355°C or higher, and more preferably 360°C or higher.

The curing shrinkage rate of the cured product is preferably 1.5% or less, more preferably 1.2% or less, and even more preferably 1.1% or less. The curing shrinkage rate is determined by measuring the density of the curable composition before curing and the density of the cured product after curing and calculating the change in density based on following equation.

$$\text{Volume shrinkage rate } r = \{(ds - dl)/dl\} \times 100$$

dl: Specific gravity of the liquid before curing. Measured using the "DA-640" density/specific gravity meter (available from Kyoto Electronics Manufacturing Co., Ltd.

ds: Specific gravity of the solid after curing. Measured in accordance with the solid specific gravity measurement method.

The curable composition can be used in various applications, such as, for example, in encapsulants, adhesives, coating agents, electrical insulation materials, laminated plates, inks, sealants, resists, composite materials, transparent substrates, transparent sheets, transparent films, optical elements, optical lenses, optical shaping, electronic paper, touch screens, solar cell substrates, optical waveguides, light guiding plates, and holographic memories.

Encapsulant

The encapsulant contains the curable composition described above. The encapsulant can be preferably used in an application to encapsulate an optical semiconductor (optical semiconductor element) in an optical semiconductor device. When the encapsulant is used, an optical semiconductor element can be encapsulated with a cured product (i.e., an encapsulating material) that excels in transparency and heat resistance and is not susceptible to curing shrinkage.

The content proportion of the curable composition in relation to the total amount (100 mass%) of the encapsulant is, for example, preferably 50 mass% or greater, more preferably 60 mass% or greater, and even more preferably 70 mass% or greater. The encapsulant may consist of only the curable composition.

Adhesive

The adhesive contains the curable composition described above. The adhesive can be used in applications to adhere and fix a member or the like to an adherend, and more specifically, the adhesive can be used in various applications that require excellent transparency and heat resistance and the ability to resist curing shrinkage, such as a die attachment paste agent for adhering and fixing an optical semiconductor element to a metal electrode in an optical semiconductor device; a lens adhesive for fixing a lens of a camera or the like to an adherend or bonding lenses together; and an optical film adhesive for fixing an optical film (such as, for example, a polarizer, a polarizer protective film, or a retardation film) to an adherend, bonding optical films together, or bonding an optical film with another film.

In particular, the adhesive can be preferably used as a die attachment paste agent (or a die bonding agent). Through the use of the adhesive as a die attachment paste agent, an optical semiconductor device can be obtained in which an optical semiconductor element is adhered to an electrode with a cured product that is excellent in transparency and heat resistance.

The content proportion of the curable composition in the total amount (100 mass%) of the adhesive is, for example, preferably 50 mass% or greater, more preferably 60 mass% or greater, and even more preferably 70 mass% or greater. The adhesive may consist of only the curable composition.

Coating Agent

The coating agent contains the curable composition described above. The coating agent can be used in various applications that require, in particular, excellent handling properties, transparency, and heat resistance.

The content proportion of the curable composition in relation to the total amount (100 mass%) of the coating agent is, for example, preferably 50 mass% or greater, more preferably 60 mass% or greater, and even more preferably 70 mass% or greater. The coating agent may consist of only the curable composition.

Optical Member

An optical member can be obtained using the cured product described above. The optical member includes the cured product of the curable composition described above. Examples of the optical member include an optical semiconductor device in which an optical semiconductor element is encapsulated by the cured product; an optical semiconductor device in which an optical semiconductor element is adhered to an electrode by the cured product; and an optical semiconductor device in which an optical semiconductor element is adhered to an electrode by the cured product, and the optical semiconductor element is encapsulated by the cured product. The optical member has a configuration in which an optical semiconductor element is encapsulated and adhered by the cured product, and thus the optical member is excellent in heat resistance and exhibits high light extraction efficiency.

Each embodiment disclosed in the present specification can be combined with any other feature disclosed in the present specification. The configurations, combinations thereof, and the like in each embodiment are exemplary, and additions, omissions, replacements, and other changes of the configurations can be appropriately made without departing from the spirit of the present disclosure. In addition, each of the inventions according to the present disclosure is not limited by the embodiments or the following examples but is limited only by the claims.

Examples

Hereinafter, an embodiment of the present disclosure will be described in more detail based on examples, but the present disclosure is not limited to these examples.

Example 1

A 20-L jacketed SUS316 reactor equipped with a stirrer was charged with 5000 g of 6-methyl-3-cyclohexenylmethyl(6'-methyl-3'-cyclohexenyl)carboxylate, and then the temperature was raised to obtain an internal temperature of 25°C. 13790 g of a 30% solution of peracetic acid in ethyl acetate was added dropwise over 6 hours, after which the mixture was aged for 3 hours. The internal temperature was maintained at 30°C during the dropwise addition and aging. In this manner, 18790 g of a crude reaction solution containing 3,4-epoxy-6-methyl-cyclohexylmethyl(3',4'-epoxy-6'-methyl) cyclohexane carboxylate was obtained.

Water Washing

The crude reaction solution obtained above was diluted 1.7 times with ethyl acetate, the diluted solution was supplied through a light liquid inlet into a centrifugal extractor and treated at a ratio of a water washing liquid to the crude reaction solution of 2, and a light liquid was obtained from a light liquid outlet at a rate of 968 g/min, and a heavy liquid was obtained from a heavy liquid outlet at a rate of 2191 g/min.

Low-Boiling Fraction Removal

A forced stirring-type thin film evaporator with a heat transfer area of 0.034 $m^2$ was charged with 100 parts by mass of the obtained light liquid, the operating pressure was maintained at 1 mmHg and the heating temperature was maintained at 170°C, and 35 parts by mass of a bottom liquid was obtained from the column bottom.

High-Boiling Fraction Removal

The bottom liquid discharged from the column bottom was charged at a charging flow rate of from 15 to 20 mL/5 min to a 15th plate counted from the bottom in a distillation column for removing high-boiling substances, with the distillation column having a column diameter of 40 mm and being formed from a perforated-plate column having an actual number of plates of 20, and the distillation column was maintained at a heating temperature of 200°C, a pressure of from 0.17 to 0.19 Torr, and a wiper rotational speed of 400 rpm. A distillate was thus distilled at a distillation flow rate of from 10 to 15 mL/min from the column top of the distillation column for removing high-boiling substances. The distillate from the column top was collected and designated as an alicyclic epoxy compound product 1 of Example 1.

Example 2

An alicyclic epoxy compound product 2 of Example 2 was obtained in the same manner as in Example 1 with the exception that the pressure in the high-boiling fraction removal was changed to a pressure from 0.075 to 0.15 Torr.

Example 3

An alicyclic epoxy compound product 3 of Example 3 was obtained in the same manner as in Example 1 with the exception that the pressure in the high-boiling fraction removal was changed to a pressure from 0.030 to 0.038 Torr.

Comparative Example 1

An alicyclic epoxy compound product 4 of Comparative Example 1 was obtained in the same manner as in Example 1 with the exception that the high-boiling fraction removal was not carried out.

Comparative Example 2

A 3,4-epoxycyclohexylmethyl(3',4'-epoxy)cyclohexane carboxylate product was used as an alicyclic epoxy compound product 5 of Comparative Example 2.

Evaluation

The alicyclic epoxy compound products obtained in the Examples and Comparative Examples were evaluated as follows. The results are shown in Table 1.

(1) $^1$H-NMR

The alicyclic epoxy compound product 1 of Example 1 was subjected to $^1$H-NMR spectrum measurements using the "JNM-ECZ400S" device (available from JEOL Ltd.), with the solvent being deuterated chloroform, and the measurement conditions including a temperature of 20°C. An $^1$H-NMR spectrum of the alicyclic epoxy compound product 1 obtained in Example 1 is presented in FIG. 1.

(2) GPC

As a pre-treatment, 0.04 g of the alicyclic epoxy compound product was dissolved in 2 g of tetrahydrofuran (THF) and then filtered through a filter (trade name "DISMIC13JP050AN", available from Toyo Roshi Kaisha, Ltd.) having a pore diameter of 0.50 μm. The obtained solution of the alicyclic epoxy compound product in THF was analyzed by GPC, and the ratio of the peak area of the component (the target alicyclic epoxy compound) having the highest peak area ratio was defined as the purity [area%] of the alicyclic epoxy compound product. The sum of the concentrations of the respective components eluted earlier than the target alicyclic epoxy compound was calculated as the high molecular weight component concentration. Note that when the shoulders of adjacent peaks overlapped, the peak areas were divided by a perpendicular line from the valley between the peaks to a base line, and the peak areas were then calculated. The GPC device used and the various conditions were as follows.

Device: HLC-8220 GPC (available from Tosoh Corporation)
Detector: Differential refractometer (RI detector)
Precolumn: TSK GUARD COLUMN SUPER HZ-L, 4.6 mm × 20mm
Column: sample side TSK-GEL SUPER HZM-N, 4.6 mm × 150 mm × 4 columns
Reference side TSK-GEL SUPER HZM-N, 6.0 mm × 150 mm × 1 column + TSK-GEL SUPER H-RC, 6.0 mm × 150 mm
Thermostatic bath temperature: 40°C
Mobile phase: THF
Moving bed flow rate: 0.35 ml/min
Sample injection volume: 10 μL
Data collection time: from 10 minutes to 26 minutes after sample

injection

(3) GC-MS

The alicyclic epoxy compound product of each example was analyzed by gas chromatography under the following measurement conditions. In addition, components contained in the alicyclic epoxy compound products were identified based on the molecular weight. The molecular weight of each detected peak was analyzed by mass spectrometry. The total content proportion of compounds (a) to (d) and the compounds having a molecular weight of 100 or less was measured using a gas chromatograph under the following conditions and calculated in terms of area%. In the Examples and Comparative Example 1, the compound (a) is the compound represented by Formula (a), the compound (b) is the compound represented by Formula (b), the compound (c) is the compound represented by Formula (c), and the compound (d) is the compound represented by Formula (d). Moreover, in Comparative Example 2, the compound (a) is a compound having a structure obtained by removing the methyl groups of two epoxycyclohexyl groups from the compound represented by Formula (a), the compound (b) is a compound having a structure obtained by removing the methyl groups of two epoxycyclohexyl groups from the compound represented by Formula (b), the compound (c) is a compound having a structure obtained by removing the methyl groups of two epoxycyclohexyl groups from the compound represented by Formula (c), and the compound (d) is a compound having a structure obtained by removing the methyl groups of two epoxycyclohexyl groups from the compound represented by Formula (d).

Measurement conditions

Measurement device: "Agilent 7890GC5977B MSD" (trade name), available from Agilent Technologies, Inc.
Column packing material: (5% phenyl)methylsiloxane
Column size: length of 15 m x inner diameter of 0.53 mmφ x film thickness of 1.5 μm
Column temperature: The temperature was increased from 100°C (at temperature increase rate of 10°C/min) to 250°C (for 15 min)
Detector: FID

(4) Hue (APHA)

The hue was evaluated by determining the Hazen color number APHA using a spectrophotometer for color and turbidity (trade name "TZ 6000", available from Nippon Denshoku Industries Co., Ltd.) and a glass cell (optical path length 33 × cell width 20 × height 55). A hue of 105 or less was determined to be good, and a hue of 15 or less was determined to be excellent.

(5) Viscosity

The viscosity of each alicyclic epoxy compound product at 25°C was measured using a digital viscometer (Model No. "DVU-EII", available from Tokyo Keiki Inc.) under conditions including a standard rotor of 1°34' × R24, a temperature of 25°C, and a rotational speed of from 0.5 to 10 rpm. A viscosity of 1300 mPa·s or less was determined to be good, and a viscosity of 1000 mPa·s or less was determined to be excellent.

Example 4

0.6 parts by mass of "SAN-AID SI-100L (trade name)" (available from Sanshin Chemical Industry Co., Ltd.) was blended as a thermal cationic catalyst into 100 parts by mass of the alicyclic epoxy compound product of each example, the mixture was stirred using a rotating/revolving-type stirring apparatus ("Awatori Rentaro AR-250 (trade name)" available from Thinky Corporation), and the mixture was further defoamed, and thereby each curable composition was obtained.

Example 5

The alicyclic epoxy compound product of each example, "RIKACID MH-700 (trade name)" (available from New Japan Chemical Co., Ltd.) as an acid anhydride curing agent, and "PX-4MP (trade name)" (available from Nippon Chemical Industrial Co.,Ltd.) as a curing accelerator were blended at amounts at which the ratio of the epoxy equivalent to the acid anhydride equivalent of the compound represented by Formula (1) in the alicyclic epoxy compound product was 100:90. The mixture was stirred using a rotating/revolving-type stirring apparatus ("Awatori Rentaro AR-250 (trade name)" available from Thinky Corporation) and further defoamed, and thereby each curable composition was obtained.

(6) Curability

The curability of the curable compositions obtained in Examples 4 and 5 was measured using a gel time measuring apparatus (trade name "Rheometer MCR302", available from Anton Paar Japan K.K.). Specifically, the curable composition (thermal cationic catalyst) of Example 4 was heated to 80°C, and the curable composition (acid anhydride

curing agent) of Example 5 was heated to 120°C, after which the curing profile was measured by the rheometer method (dynamic viscoelasticity evaluation) to measure the temperature curve of the loss modulus at a constant frequency, and the point of intersection between two elastic modulus curves obtained by measuring the G' (storage modulus) and the G" (loss modulus) was defined as the gelling point. In addition, the point at which the set temperature increase (80°C or 120°C) was reached was set as a starting point, and the time to reach the gelling point was evaluated as the thermal gel time. In Example 4, a thermal gel time of 1100 seconds or less was determined to be good, and a thermal gel time of 1050 seconds or less was determined to be excellent. In Example 5, a thermal gel time of 5000 seconds or less was determined to be good, and a thermal gel time of 3000 seconds or less was determined to be excellent.

Example 6

Molds were filled with each of the curable compositions obtained in Examples 4 and 5 and then heated in a resin curing oven at 120°C for 5 hours, and respective cured products were thereby obtained.

(7) Curing Shrinkage

The density before and after curing of the cured product obtained from each curable composition of Example 4 was measured in accordance with a density measurement method (JIS K5600 2-4), and the curing shrinkage rate (volume shrinkage rate) was determined from the change in density based on the following equation. A curing shrinkage rate of 1.5% or less was determined to be good.

$$\text{Volume shrinkage rate r} = \{(ds - dl)/dl\} \times 100$$

dl: Specific gravity of the liquid before curing. Measured using the "DA-640" density/specific gravity meter (available from Kyoto Electronics Manufacturing Co., Ltd.

ds: Specific gravity of the solid after curing. Measured in accordance with the solid specific gravity measurement method.

(8) Light Transmittance

The light transmittance (in the thickness direction) for light with a wavelength of 450 nm of the cured product (thickness: 3 mm) obtained in Example 6 was measured using a spectrophotometer ("UV-2450 (trade name)", 10 mm square quartz cell, thickness of 10 mm, available from Shimadzu Corporation). In Example 4, a light transmittance of 80% or greater was determined to be good, and a light transmittance of 85% or greater was determined to be excellent. In Example 5, a light transmittance of 88% or greater was determined to be good, and a light transmittance of 90% or greater was determined to be excellent.

(9) Glass Transition Temperature (Tg)

The glass transition temperature of each cured product obtained in Example 6 was determined under the conditions below. In Example 4, a glass transition temperature of 175°C or higher was determined to be good, and a glass transition temperature of 200°C or higher was determined to be excellent. In Example 5, a glass transition temperature of 180°C or higher was determined to be good, and a glass transition temperature of 190°C or higher was determined to be excellent.

Sample: 4 mm in length x 5 mm in width x 10 mm in thickness

Measurement apparatus: thermomechanical measurement apparatus (TMA), "TMA/SS6000 (trade name)", available from Seiko Instruments Inc.
Measurement mode: compression (needle penetration), constant load measurement
Measurement temperature: from 25°C to 300°C
Rate of temperature increase: 5°C/min

(10) Weight Loss Temperature (TG/DTA)

The 5% weight loss temperature (Td5) and the 10% weight loss temperature (Td10) were determined under the following conditions for the cured products obtained from each curable composition of Example 4. A Td5 of 325°C or higher was determined to be good, and a Td5 of 335°C or higher was determined to be excellent. A Td10 of 355°C or higher was determined to be good, and a Td10 of 360°C or higher was determined to be excellent.

Sample: 5 to 10 μg
Measurement apparatus: "STA/7200 (trade name)", available from Hitachi High-Technologies Corporation

(11) Light Transmittance Retention Rate

Each cured product (3 mm thick) obtained in Example 6 was (i) irradiated with ultraviolet rays at an intensity 10 mW/cm$^2$ for 500 hours while being heated at 120°C or (ii) subjected to a heating treatment at 120°C, after which the light transmittance (%) of ultraviolet rays (wavelength of 450 nm) was measured using a spectrophotometer (product name "UV-2450", available from Shimadzu Corporation). In addition, the value of the light transmittance after the treatment (the retention rate of the light transmittance of the cured product) with respect to the light transmittance before the treatment (the light transmittance described in (8) above) was evaluated as the light transmittance retention rate (light resistance) (%). A larger value of the transmittance retention rate indicates more excellent light resistance of the cured product with respect to ultraviolet rays.

[Table 1]

| | | Comparative Example 1 | Example 1 | Example 2 | Example 3 | Comparative Example 2 |
|---|---|---|---|---|---|---|
| Epoxy compound product | Purity [%] | 83.2 | 90.0 | 92.1 | 98.3 | 96.7 |
| | Proportion [wt%] of compound (a) | 5.07 | 2.84 | 1.93 | 0.43 | 1.03 |
| | Proportion [wt%] of compound (b) | 0.10 | 0.02 | 0.02 | 0.02 | 0.02 |
| | Proportion [wt%] of compound (c) | 6.05 | 3.39 | 2.31 | 0.51 | 1.23 |
| | Proportion [wt%] of compound (d) | 3.94 | 2.21 | 1.52 | 0.32 | 0.80 |
| | Proportion [wt%] of compounds having molecular weight of 100 or less | 1.64 | 1.03 | 0.74 | 0.42 | 0.41 |
| | Viscosity | 1722 | 1283 | 1146 | 707 | 245 |
| | Hue | 164 | 104 | 103 | 11 | 10 |

(continued)

| | | | Comparative Example 1 | Example 1 | Example 2 | Example 3 | Comparative Example 2 |
|---|---|---|---|---|---|---|---|
| Cured product | Light transmittance (thermal cationic) [%] | | 79.6 | 82.7 | 83.4 | 86.7 | 85.4 |
| | Light transmittance (acid anhydride) [%] | | 87.0 | 88.3 | 88.6 | 90.7 | 90.6 |
| | Tg (thermal cationic) [°C] | | 161 | 179 | 186 | 203 | 210 |
| | Tg (acid anhydride) [°C] | | 176 | 183 | 184 | 191 | 189 |
| | Td5 [°C] | | 321 | 328 | 330 | 335 | 357 |
| | Td10 [°C] | | 350 | 356 | 358 | 364 | 374 |
| | Curing shrinkage [%] | | 1.0 | 1.1 | 1.1 | 1.1 | 2.7 |
| | Thermal gel time (acid anhydride) [seconds] | | 1112 | 1083 | 1082 | 1040 | 1564 |
| | Thermal gel time (thermal cationic) [seconds] | | 6070 | 4500 | 3868 | 2820 | 3172 |
| | 450 nm light transmittance retention rate (acid anhydride) [%] | 120°C UV | 79 | 79 | 79 | 83 | 83 |
| | | 120°C | 92 | 92 | 92 | 96 | 93 |
| | 450 nm light transmittance retention rate (thermal cationic) [%] | 120°C UV | 18 | 20 | 20 | 20 | 40 |
| | | 120°C | 59 | 61 | 61 | 63 | 80 |

As shown in Table 1, the alicyclic epoxy compound products of the Examples were evaluated to have low viscosity, good hue, and excellent transparency as compared with the products having low purity. In addition, evaluations of the cured products indicated that the cured products exhibited high light transmittance, excellent transparency, a high retention rate, a high Tg, and excellent heat resistance. Furthermore, the evaluations indicated that the alicyclic epoxy compound products of the Examples exhibited less curing shrinkage than the products of the other alicyclic epoxy compounds.

Example 7

2 parts by mass of a thermal cationic catalyst ("CPI-210S (trade name)" (available from San-Apro Ltd.) was blended as a curing catalyst into 100 parts by mass of the alicyclic epoxy compound product 1 obtained in Example 1, the mixture was stirred using a rotating/revolving-type stirring apparatus ("Awatori Rentaro AR-250 (trade name)" available from Thinky Corporation), and the mixture was further defoamed, and thereby a curable composition was obtained. A mold was filled with the curable composition, irradiated with ultraviolet rays at an intensity of 100 $mW/cm^2$ for 30 seconds, and then heated in a resin curing oven at 150°C for 30 minutes to obtain a cured product.

Examples 8 to 16

Curable compositions and cured products were prepared in the same manner as in Example 7 with the exception that the components and the contents were as shown in Table 2.

Evaluation

The curable compositions of the Examples were evaluated for curability and for the Tg and weight loss temperature of the cured products. The results are shown in Table 2. The methods for evaluating the Tg and weight

loss temperature were the same as in Example 1. The method for evaluating curability was as follows.

(12) Curability

The curability of each of the curable compositions obtained in Examples 8 to 16 was measured using the "DSC6220" device available from Seiko Instruments Inc. (SII). Specifically, at 30°C, the curable compositions were irradiated at 100 mW for 30 seconds by a light source having a wavelength of 365 nm, and the exothermic peak intensity was calculated. A higher peak intensity indicates more excellent curability.

[Table 2]

| | | Example 7 | Example 8 | Example 9 | Example 10 | Example 11 | Example 12 | Example 13 | Example 14 | Example 15 | Example 16 |
|---|---|---|---|---|---|---|---|---|---|---|---|
| Epoxy compound product | Alicyclic epoxy compound product 1 | 100 | 95 | 90 | 95 | 90 | | | | | |
| | Alicyclic epoxy compound product 2 | | | | | | | | | | |
| | Alicyclic epoxy compound product 3 | | | | | | 100 | 95 | 90 | 95 | 90 |
| Other epoxy compound | (3,4,3',4'-diepoxy) bicyclohexyl | | 5 | 10 | | | | 5 | 10 | | |
| Oxetane compound | 3-ethyl-3{[(3-ethyloxetan-3-yl)methoxy] methyl} oxetane | | | | 5 | 10 | | | | 5 | 10 |
| Curing catalyst | CPI-210S | 2 | 2 | 2 | 2 | 2 | 2 | 2 | 2 | 2 | 2 |
| Reactivity | Peak intensity [mW] | 13 | 17 | 20 | 20 | 27 | 16 | 19 | 22 | 24 | 31 |
| Cured product | Tg [°C] | 150 | 156 | 165 | 153 | 163 | 158 | 162 | 169 | 160 | 166 |
| | Td5 [°C] | 228 | 230 | 231 | 233 | 235 | 231 | 232 | 232 | 234 | 237 |
| | Td10 [°C] | 248 | 249 | 249 | 251 | 254 | 250 | 251 | 249 | 253 | 256 |

Hereinafter, variations of the invention according to the present disclosure will be described.

[Supplement 1] An epoxy compound product, wherein

a purity of a compound represented by Formula (1) below is 90% or greater; and

a total content proportion of a compound represented by Formula (a) below, a compound represented by Formula (b) below, a compound represented by Formula (c) below, and a compound represented by Formula (d) below is 10 mass% or less.

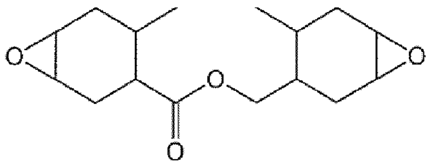

(1)

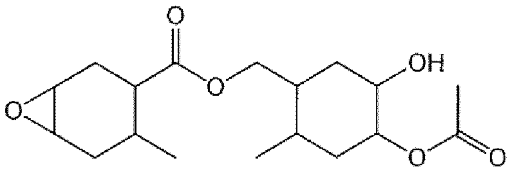

(a)

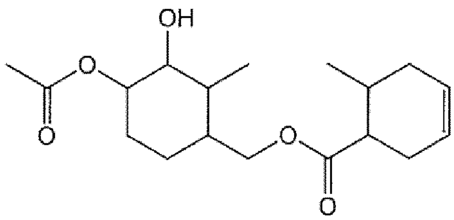

(b)

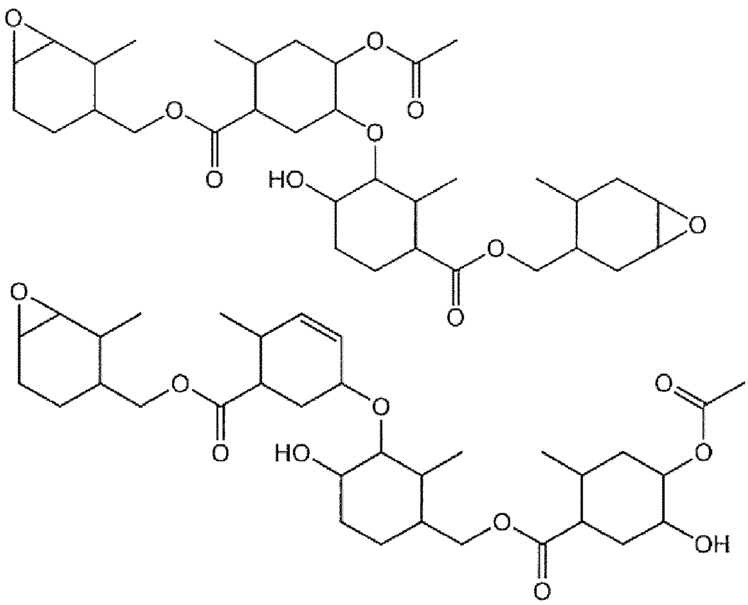

(c)

(d)

[Supplement 2] The epoxy compound product according to supplement 1, wherein the epoxy compound product has a Hazen color number of 105 or less.

[Supplement 3] The epoxy compound product according to supplement 1 or 2, wherein the total content proportion of the compound represented by Formula (a) below, the compound represented by Formula (b) below, the compound represented by Formula (c) below, and the compound represented by Formula (d) below is 0.1 mass% or greater.

[Supplement 4] A curable composition containing: the epoxy compound product described in any one of

supplements 1 to 3; and a curing agent and/or a curing catalyst.

[Supplement 5] A curable composition containing: the epoxy compound product described in any one of supplements 1 to 3; and another epoxy compound and/or an oxetane compound.

[Supplement 6] The curable composition described in supplement 4 or 5, wherein the curable composition is an adhesive, an encapsulant, or a coating agent.

[Supplement 7] A cured product of the curable composition described in any one of supplements 4 to 6.

[Supplement 8] An optical member provided with the cured product described in supplement 7.

[Supplement 9] A method for producing the epoxy compound product described in supplement 1 or 2, wherein the epoxy compound product is produced by carrying out epoxidation, low-boiling fraction removal, and high-boiling fraction removal;

the epoxidation includes reacting 6-methyl-3-cyclohexenylmethyl(6'-methyl-3'-cyclohexenyl)carboxylate with an organic peracid to produce a reaction product;
the low-boiling fraction removal includes subjecting the reaction product to a low-boiling fraction removal treatment; and
the high-boiling fraction removal includes subjecting the reaction product to a high-boiling fraction removal treatment through thin film evaporation

## Claims

**1.** An epoxy compound product, wherein

a purity of a compound represented by Formula (1) is 90% or greater; and
a total content proportion of a compound represented by Formula (a), a compound represented by Formula (b), a compound represented by Formula (c), and a compound represented by Formula (d) is 10 mass% or less,

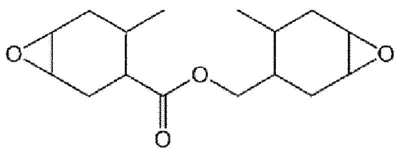

(1)

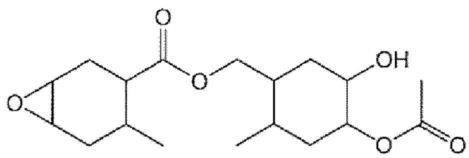

(a)

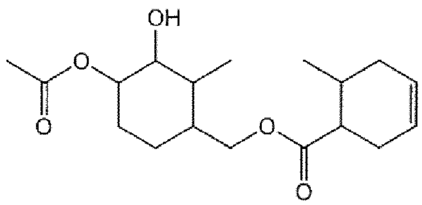

(b)

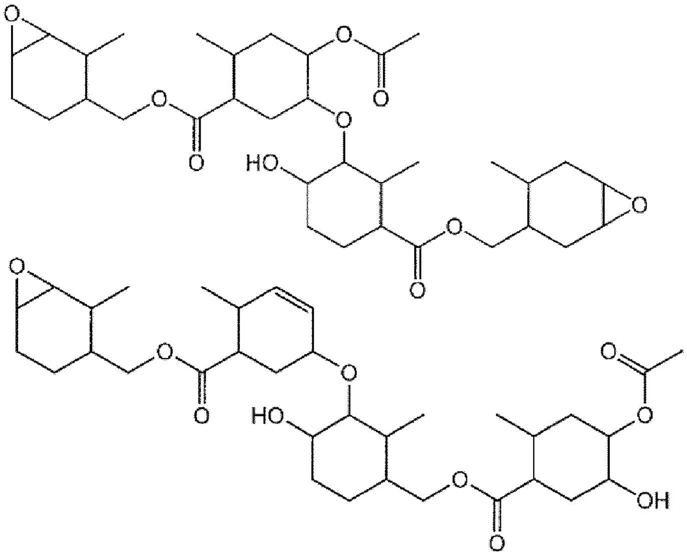

(c)

(d)

2. The epoxy compound product according to claim 1, wherein the epoxy compound product has a Hazen color number of 105 or less.

3. The epoxy compound product according to claim 1, wherein the total content proportion of the compound represented by Formula (a), the compound represented by Formula (b), the compound represented by Formula (c), and the compound represented by Formula (d) is 0.1 mass% or greater.

4. A curable composition comprising: the epoxy compound product described in claim 1; and a curing agent and/or a curing catalyst.

5. A curable composition comprising: the epoxy compound product described in claim 1; and another epoxy compound and/or an oxetane compound.

6. The curable composition according to claim 4 or 5, wherein the curable composition is an adhesive, an encapsulant, or a coating agent.

7. A cured product of the curable composition described in claim 4 or 5.

8. An optical member comprising the cured product described in claim 7.

9. A method for producing the epoxy compound product described in claim 1 or 2, wherein the epoxy compound product is produced by carrying out epoxidation, low-boiling fraction removal, and high-boiling fraction removal;

   the epoxidation includes reacting 6-methyl-3-cyclohexenylmethyl(6'-methyl-3'-cyclohexenyl)carboxylate with an organic peracid to produce a reaction product;
   the low-boiling fraction removal includes subjecting the reaction product to a low-boiling fraction removal treatment; and
   the high-boiling fraction removal: subjecting the reaction product to a high-boiling fraction removal treatment through thin film evaporation.

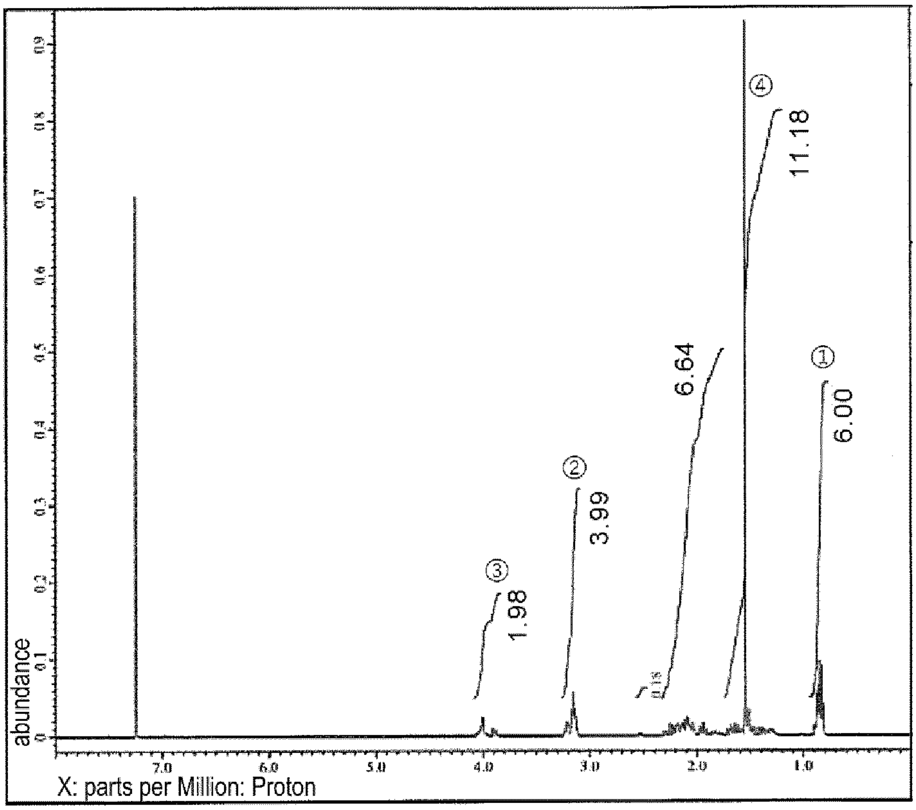
abundance
X: parts per Million: Proton
7.0
6.0
5.0
4.0
3.0
2.0
1.0
①
6.00
②
3.99
③
1.98
④
11.18
6.64

FIG. 1

## INTERNATIONAL SEARCH REPORT

International application No.
**PCT/JP2023/038956**

### A. CLASSIFICATION OF SUBJECT MATTER

***C08G 59/24***(2006.01)i; ***C07D 303/44***(2006.01)i; ***C09D 7/63***(2018.01)i; ***C09D 163/00***(2006.01)i; ***C09J 11/06***(2006.01)i; ***C09J 163/00***(2006.01)i
FI: C08G59/24; C09J163/00; C09J11/06; C09D163/00; C09D7/63; C07D303/44

According to International Patent Classification (IPC) or to both national classification and IPC

### B. FIELDS SEARCHED

Minimum documentation searched (classification system followed by classification symbols)

C08G59/00-59/72; C07D303/44; C09D7/63; C09D163/00; C09J11/06; C09J163/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X<br>Y | US 2999827 A (UNION CARBIDE CORPORATION) 12 September 1961 (1961-09-12) column 10, line 14 to column 14, line 29, examples 1-9, 17-72, claims | 1-4, 6-7<br>5, 8-9 |
| Y | WO 2019/138988 A1 (DAICEL CORPORATION) 18 July 2019 (2019-07-18) claims, paragraphs [0024]-[0029], [0035]-[0083], [0122], examples 1-3 | 5, 8-9 |
| A | WO 2006/073093 A1 (DAICEL CHEMICAL INDUSTRIES, LIMITED) 13 July 2006 (2006-07-13) claims | 1-9 |
| A | WO 2005/090325 A1 (DAICEL CHEMICAL INDUSTRIES, LIMITED) 29 September 2005 (2005-09-29) claims | 1-9 |
| A | WO 2020/213526 A1 (DAICEL CORPORATION) 22 October 2020 (2020-10-22) claims | 1-9 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 January 2024** | **23 January 2024** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br><br>Telephone No. |

Form PCT/ISA/210 (second sheet) (July 2022)

**INTERNATIONAL SEARCH REPORT**
**Information on patent family members**

International application No.
**PCT/JP2023/038956**

| Patent document cited in search report | Publication date (day/month/year) | Patent family member(s) | Publication date (day/month/year) |
|---|---|---|---|
| US 2999827 A | 12 September 1961 | GB 896971 A | |
| | | FR 1248712 A | |
| | | BE 586666 A | |
| | | CH 429190 A | |
| | | NL 247540 A | |
| | | NL 126205 C | |
| WO 2019/138988 A1 | 18 July 2019 | US 2021/0189058 A1 claims, paragraphs [0029]-[0032], [0039]-[0095], [0134], examples 1-3 | |
| | | CN 111587267 A | |
| | | TW 201936690 A | |
| WO 2006/073093 A1 | 13 July 2006 | US 2008/0045729 A1 claims | |
| | | EP 1834949 A1 | |
| | | CN 101087772 A | |
| | | KR 10-2007-0108370 A | |
| | | TW 200631948 A | |
| WO 2005/090325 A1 | 29 September 2005 | US 2007/0179256 A1 claims | |
| | | EP 1726588 A1 | |
| | | CN 1934098 A | |
| | | KR 10-2007-0005629 A | |
| | | TW 200604188 A | |
| WO 2020/213526 A1 | 22 October 2020 | US 2022/0194913 A1 claims | |
| | | JP 2020-176227 A | |
| | | CN 113728031 A | |
| | | TW 202104199 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 2022177527 A
- WO 2019138988 A
- US 2890194 A